# EUROPÄISCHE PATENTSCHRIFT

(11) **EP 0 560 141 B1**
(45) Veröffentlichungstag und Bekanntmachung des Hinweises auf die Patenterteilung: **30.10.1996**
(21) Anmeldenummer: 93102993.8
(22) Anmeldetag: 25.02.1993
(51) Int. Cl.: A61F 2/44

(54) **Bandscheibenendoprothese**
Intervertebral disc endoprosthesis
Endoprothèse pour disques intervertébraux

(30) Priorität: 13.03.1992 DE 4208116
(43) Veröffentlichungstag der Anmeldung: 15.09.1993
(73) Patentinhaber: Waldemar Link (GmbH & Co.), D-22315 Hamburg (DE)
(72) Erfinder: Büttner-Janz, Karin, Dr., O-1144 Berlin (DE); Keller, Arnold, W-2061 Kayhude (DE); Lemaire, Jean-Philippe, Dr., F-21121 Fontaine les Dijon (FR)
(74) Vertreter: Glawe, Delfs, Moll & Partner

(56) Entgegenhaltungen:
- DE-C- 3 023 353
- US-A- 4 759 766

## Beschreibung

Die Erfindung betrifft eine Bandscheibenendoprothese mit zwei Abschlußplatten, die mit den Endplatten der zugehörigen Wirbelkörper zu verbinden sind, und einem Prothesenkern, der mit mindestens einer Abschlußplatte über eine eine Schwenkbewegung gestattende Gelenkfläche zusammenwirkt.

Bei bekannten Bandscheibenendoprothesen dieser Art (EP-A-0 176 728, FR-A-26 59 226, DE-A-28 04 936) ist die Gelenkfläche sphärisch. Die durch die Prothese miteinander verbundenen Wirbelkörper können daher nicht nur Beugebewegungen in der Medianebene und der Frontalebene, sondern auch Rotationsbewegungen um die Hochachse frei durchführen. Diese Rotationsbewegung wird auch nicht durch Anschläge von Abschlußplattenteilen aneineinander gehindert. - Bei einer anderen bekannten Bandscheibenendoprothese (DE-G-30 23 353) wird hingegen eine Rotationsbewegung durch Abschlußplattenanschläge aneinander unmöglich gemacht; eine Schwenkbewegung ist lediglich in der Medianebene möglich. Dies hat nicht nur den Nachteil, daß an den Abschlußplatten Teile aus gleichem Material mit entsprechend hohem Abrieb und hoher Reibung aufeinander gleiten, sondern daß auch bei einer Rotationsbewegung des Körpers auf die Verankerung der Prothese an den zugehörigen Wirbelkörpern hohe Kräfte wirken, die im Hinblick auf eine dauerhafte Fixation der Prothesenteile an den Wirbelkörpern nachteilig sind. Jedoch hat auch die freie Drehbeweglichkeit der zuvor erwähnten Prothesen Nachteile in sofern, als der notwendige Drehwiderstand, der von der natürlichen Bandscheibe dank deren Faserbeschaffenheit und -anordnung geleistet werden kann, mindestens bis zur Bildung ausreichenden Narbengewebes entfällt und daher die Wirbelbogengelenke überlastet werden können, was zu Beschwerden führen kann. Erwünscht ist deshalb eine Bandscheibenendoprothese, die der Verdrehung um die Hochachse einen Widerstand entgegensetzt, ohne daß dies durch Anschläge an den Abschlußplatten erzielt wird.

Die erfindungsgemäße Lösung besteht darin, daß die Gelenkfläche im mittigen Sagittalschnitt und im mittigen Frontalschnitt Krümmungsbögen von unterschiedlichem Radius bildet. Vorzugsweise sind die Krümmungsradien im Sagittalschnitt kleiner als im Frontalschnitt. Die Krümmungsradien in den zueinander quer stehenden Hauptrichtungen unterscheiden sich zweckmäßigerweise um den Faktor 1,2 bis 2,5, vorzugsweise 1,5 bis 2. Sie sind zweckmäßigerweise in den Hauptschnittebenen Kreisbögen oder der Kreisbogenform angenähert.

Die Wirkung dieser Maßnahme besteht darin, daß eine Rotation der Prothesenteile um die Hochachse zwar möglich ist, daß aber die Prothese dabei infolge des Aufgleitens der seitlichen schrägen Flanken der Gleitflächen aufeinander auseinandergespreizt wird, wodurch aufgrund der Gewichtsbelastung ein rückführendes Moment entsteht. Umgekehrt könnte man auch sagen, daß die Rotationsbewegung der Prothese um die Hochachse sanft gebremst wird und die Prothese eine Tendenz hat, zur neutralen Stellung zurückzukehren.

In manchen Fällen kann es trotz des erfindungsgemäß erzeugten Widerstands gegen eine Rotation der Prothese um die Hochachse zweckmäßig sein, den maximal verfügbaren Rotationswinkel durch Anschläge zu begrenzen, die zwischen den Prothesenplatten oder besser zwischen dem Prothesenkern und den damit über Gelenkflächen zusammenwirkenden Prothesenplatten wirken können. Diese Anschläge werden so angeordnet, daß sie im allgemeinen nicht in Funktion treten, weil innerhalb des normalen Rotationsbereichs der durch die erfindungsgemäße Formgebung der Gleitflächen erzeugte Rotationswiderstand ausreicht. Sie sollen lediglich eine Rotation solchen Ausmaßes ausschließen, daß dadurch Schäden am Wirbelapparat oder an der Prothese verursacht werden könnten. Im allgemeinen vermeidet man Anschläge zwischen Prothesenteilen, weil bei heftigem Anschlag unter Körperschwung Kräfte entstehen können, die den Verbund zwischen dem Prothesenteil und dem Knochen gefährden können. Diese Gefahr besteht im vorliegenden Fall nicht, weil der Körperschwung durch den Rotationswiderstand der Gleitflächen hinreichend geschwächt worden ist, bevor die Anschläge erreicht werden.

Bei einer bevorzugten Ausführungsform werden die Anschläge in die Gleitflächen des Prothesenkerns und der damit zusammenwirkenden Prothesenplatte(n) eingearbeitet. Diese können zweckmäßigerweise die Form einer Nut in der einen Fläche und einer Feder in der anderen Fläche annehmen, wobei deren Kontur so gewählt ist, daß der gewünschte Rotationsbereich ebenso gewährleistet ist wie eine hinreichende Beugung nach vorne und zu beiden Seiten. Zwar ist es bekannt (US-A-4 759 766, zur Patentfemilie der weiter oben erwähnten EP-A-0 176 728 gehörig), die Prothesenplatten und den Prothesenkern mit zusammenwirkenden Vorsprüngen und Nuten zu versehen. Jedoch ist deren Form nicht zur Bildung eines Anschlags gegen Rotationsbewegung geeignet; dies ist auch nicht erforderlich, weil die Gelenkflächen zylindrisch sind und keine Rotationsmöglichkeit bieten.

Zusätzlich zu oder statt der Nut-Feder-Verbindung kann die Rotationsbegrenzung auch bewirkt werden durch einen vorzugsweise umlaufenden Kragen an einem der beiden Teile und einen damit zusammenwirkenden Rand am anderen Teil. Solche zusammenwirkenden Kragen und Ränder an dem Prothesenkern und den Prothesenplatten sind bei kreisrund begrenzten Prothesenteile an sich bekannt (EP-A 0 176 728, Fig. 1 bis 3), um die Teile gegenüber seitlicher Relativbewegung zusammenzuhalten. Bei ovaler Grundform der Teile vermögen sie auch deren Rotation um die Hochachse zu begrenzen.

Die Erfindung wird im folgenden näher unter Bezugnahme auf die Zeichnung erläutert, die vorteilhafte Ausführungsbeispiele veranschaulicht. Darin zeigen:
- Fig. 1: einen mittleren Frontalschnitt durch die Prothese in deren Neutralstellung,
- Fig. 2: einen der Fig. 1 entsprechenden Schnitt bei seitlicher Beugung,
- Fig. 3: einen Medianschnitt durch die Prothese,
- Fig. 4: eine der Fig. 3 entsprechende Darstellung bei Beugung nach vorne,

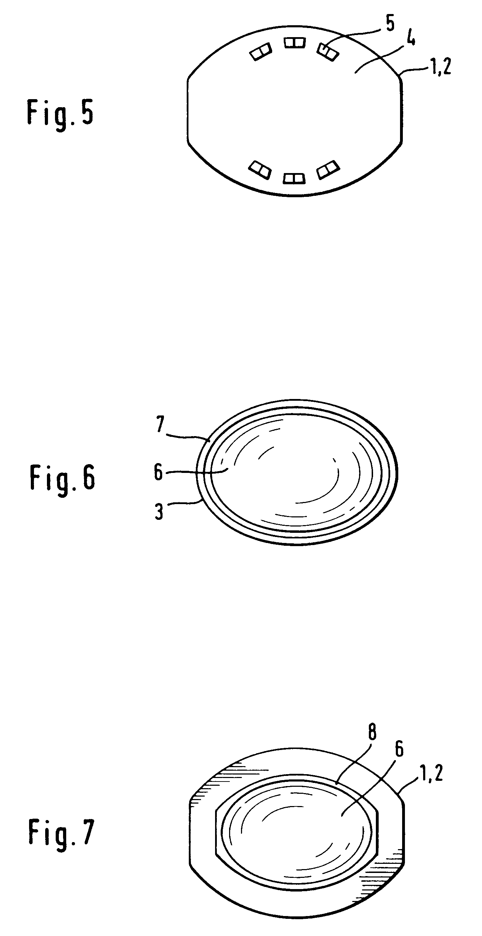

- Fig. 5: eine Draufsicht auf die Außenfläche der Prothesenplatten,
- Fig. 6: eine Draufsicht auf den Prothesenkern,
- Fig. 7: eine Draufsicht auf die Innenseite der Prothesenplatten, jeweils von einer ersten Ausführungsform der Erfindung,
- Fig. 8 bis 11: Darstellungen entsprechend den Fig. 1, 3, 6 und 7 einer zweiten Ausführungsform,
- Fig. 12 und 13: Darstellung entsprechend den Fig. 1 und 3 einer dritten Ausführungsform,
- Fig. 14 und 15: zwei der Fig. 1 entsprechende Darstellungen einer vierten und einer fünften Ausführungsform der Erfindung.

Die dargestellte Bandscheibenendoprothese besteht aus den Prothesenplatten, nämlich der Grundplatte 1 und der Deckplatte 2, und dem Prothesenkern 3. Die Prothesenplatten bestehen aus Metall. Ihre Deckflächen 4, die sich an die ihnen zugewendeten Deckflächen der Wirbelkörper anlegen, sind mit Zähnen 5 versehen, die in die Wirbelkörper eindringen und dadurch eine Fixation der Prothese gegenüber den Wirbelkörpern bewirken.

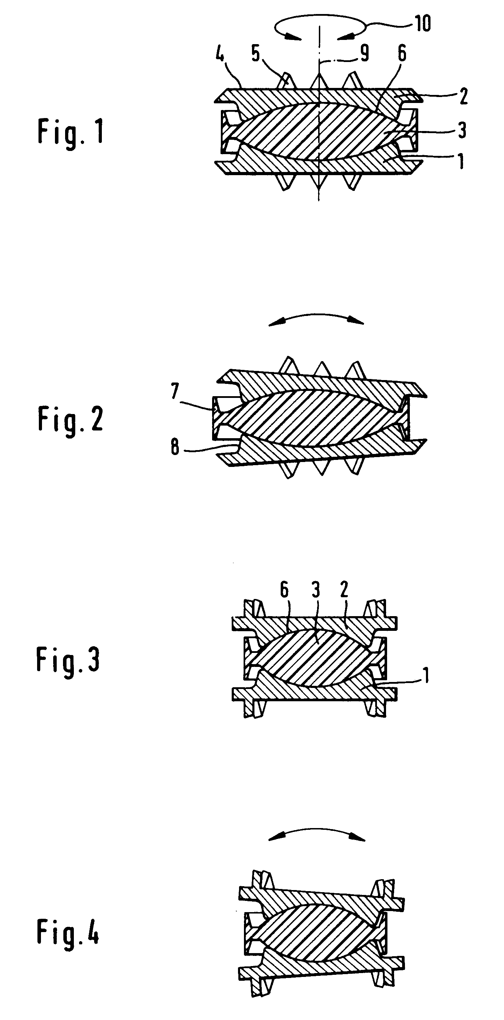

Der Prothesenkern 3 besteht aus Kunststoff mit guten Gleiteigenschaften, insbesondere hochdichtem Polyethylen. Die Prothesenplatten und der Prothesenkern bilden Gelenkflächen 6, die im Neutralzustand (Fig. 1 und 3) kongruent sind. Im mittigen Frontalschnitt (Fig. 1) stellen sie ebenso Kreisbögen dar wie im Medianschnitt (Fig. 3). In allen sonstigen Sagittal- und Frontalschnitten bilden sie vorzugsweise Kreisbögen oder sind sie Kreisbögen angenähert. Dadurch ergeben sich die in Fig. 2 und 4 veranschaulichten Möglichkeiten einer seitlichen und frontalen Beugebewegung, die bei schräger Beugung auch kombiniert auftreten können. Der Prothesenkern 3 ist mit einem Kragen 7 versehen, der mit dem Rand 8 der Prothesenplatten 1, 2 zusammenwirkt, um die seitliche Relativbewegung des Prothesenkerns gegenüber den Prothesenplatten zu beschränken, wie man in Fig. 2, rechte Seite, gut erkennt. Insoweit kann die Prothese als bekannt betrachtet werden.

Erfindungsgemäß sind die Krümmungsradien der Gelenkflächen 6 im Sagittalschnitt und Frontalschnitt unterschiedlich, nämlich im Sagittalschnitt (Fig. 3) kleiner als im Frontalschnitt (Fig. 1). Während bei sphärischer Gestaltung der Gleitflächen deren Kongruenz auch dann noch erhalten bleibt, wenn die Prothesenplatten sich gegenüber dem Prothesenkern (im Sinne des Pfeils 10) um die Hochachse 9 verdrehen, ist die Kongruenz bei der erfindungsgemäßen Gelenkflächenkonfiguration in den Schnittebenen, die von den Hauptebenen abweichen, nicht mehr vorhanden. In diesen äußeren Schnittebenen steigt der Prothesenkern bei der Rotation an den Flanken der Gelenkflächen der Prothesenplatten hoch. Dadurch werden die Prothesenplatten entgegen dem auf ihnen lastenden Körpergewicht schraubenförmig auseinandergezwungen. Das Körpergewicht verursacht demzufolge ein gegenwirkendes Moment, das die Prothesenplatten in die Neutralstellung zurückzudrehen sucht. Der Verdrehung der Prothesenplatten wirkt ein Widerstand entgegen, dessen Größe durch die Steilheit der zusammenwirkenden Flanken der Gelenkflächen der Prothesenplatten und des Prothesenkerns bestimmt wird. Durch die Bemessung dieser Flanken läßt sich der Widerstand somit in geeigneter Weise bemessen. Er wird zweckmäßigerweise so bemessen, daß er keinesfalls die zwischen den Prothesenplatten und den Wirbelkörpern übertragbaren Verankerungskräfte übersteigt.

Bei einem gewissen Verdrehwinkel kommt der Kragen 7 des Prothesenkerns mit dem Rand 8 der Prothesenplatten auch dann in Eingriff, wenn die Symmetrieachsen 9 sämtlicher Komponenten miteinander fluchten. Dadurch ist ein Anschlag gegen eine zu große Verdrehung gegeben. Durch die Bemessung des Abstands des Kragens von den Gelenkflächen bzw. von dem Rand 8 der Neutralstellung kann leicht der Verdrehwinkel bemessen werden, bei welchem die Anschlagfunktion eintritt. In diesem Zusammenhang sei vermerkt, daß der Verlauf des Kragens 7 bzw. des Randes 8 nicht unbedingt parallel sein muß zum Verlauf der äußeren Begrenzung der Gelenkflächen 6.

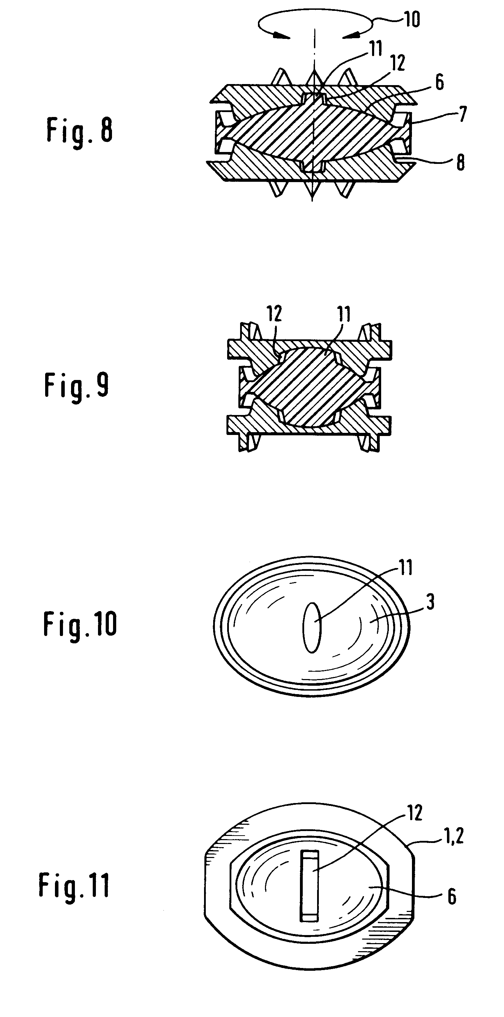

Die zweite Ausführungsform gemäß Fig. 8 bis 11 stimmt mit der ersten Ausführungsform überein, soweit nicht im folgenden anders beschrieben.

Für den Fall, daß die durch den Kragen 7 und den Rand 8 gegebene Begrenzung der Rotationsbewegung 10 nicht ausreicht, ist der Prothesenkern mit einer in der Medianebene verlaufenden Rippe 11 versehen, die in eine entsprechend verlaufende Nut 12 in der zugeordneten Gelenkfläche der Prothesenplatte(n) eingreift. Die Umrisse der Rippe 11 und der Nut 12 in Draufsicht (Fig. 10 und 11) unterscheiden sich ein wenig. Nicht nur ist die Rippe 11 im Medianschnitt und im Frontalschnitt ein wenig kürzer als die Nutweite, damit die Beugungsbewegung gemäß Fig. 2 und 4 durchgeführt werden können, sondern vor allem auch ist die Rippe 11 an ihren Enden schmaler als im Mittelbereich und als die Nut, so daß sie eine gewisse Drehbewegung innerhalb der Nut 12 durchführen kann. Diese Rotationsbewegung wird erst dann beendet, wenn die schmaleren Enden der Rippe 11 an den Seitenwänden der Nute 12 anschlagen. Falls - wie in den dargestellten Beispielen vorausgesetzt - die Prothese mit paarigen Gleitflächen oberhalb und unterhalb des Prothesenkerns versehen ist, müssen selbstverständlich zu beiden Prothesenplatten hin Anschläge des Prothesenkerns vorgesehen sein. Wenn der Prothesenkern hingegen mit einer Prothesenplatte fest verbunden ist, genügen derartige Anschläge an lediglich einer Seite.

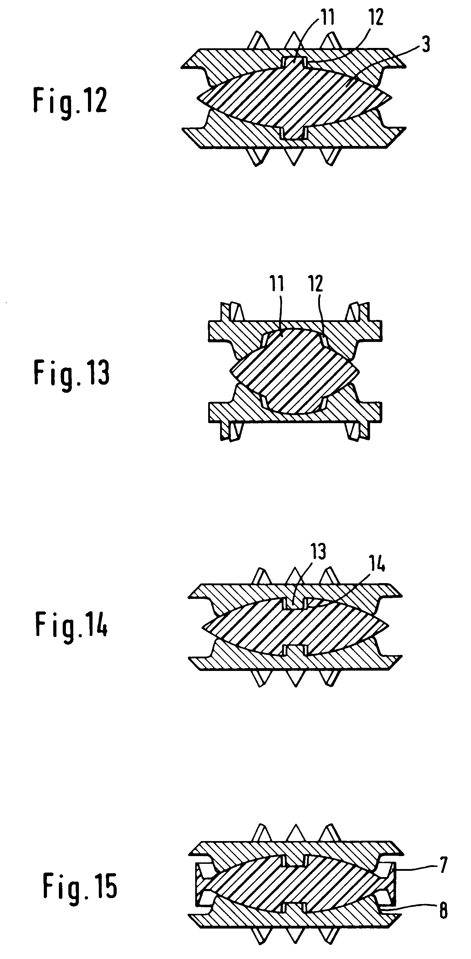

Da die Rippe 11 im Zusammenwirken mit der Nut 12 nicht nur die Rotationsbewegung, sondern auch die Beugebewegungen der Prothese begrenzt, ist der Kragen 7 am Prothesenkern nicht erforderlich, wie die Ausführung gemäß Fig. 12 und 13 zeigt, die ansonsten mit derjenigen gemäß Fig. 8 bis 11 übereinstimmt.

Während in der zweiten und dritten Ausführung (Fig. 8 bis 13) die Rippe am Prothesenkern und die damit zusammenwirkende Nut an den Prothesenplatten vorgesehen war, sind die Verhältnisse bei der Ausführungsform gemäß Fig. 14 umgekehrt; die Rippe 13 an den Prothesenplatten ragt jeweils in eine Nut 14 am Prothesenkern. Auch in diesem Fall hat die Rippe 13 die in Fig. 10 dargestellte, nach den Enden zu sich verjüngende Form, während die Nut 14 parallelflankig ist.

Die Ausführung gemäß Fig. 14 läßt sich sowohl ohne als auch mit Kragen am Prothesenkern benutzen. Während die Ausführung gemäß Fig. 14 die Version ohne Kragen darstellt, veranschaulicht Fig. 15 die Alternative mit Kragen 7 am Prothesenkern 3.

## Patentansprüche

1. Bandscheibenendoprothese mit zwei Prothesenplatten (1, 2), die mit den Endplatten der zugehörigen Wirbelkörper zu verbinden sind, und einem Prothesenkern (3), der mit mindestens einer Prothesenplatte (1, 2) über eine eine Rotationsbewegung (10) um die Hochachse (9) gestattende Gelenkfläche (6) zusammenwirkt, dadurch gekennzeichnet, daß die Gelenkfläche (6) im mittigen Medianschnitt und im mittigen Frontalschnitt Krümmungsbögen von unterschiedlichem mittlerem Radius bildet.

2. Bandscheibenendoprothese nach Anspruch 1, dadurch gekennzeichnet, daß der Krümmungsradius im Medianschnitt (Fig. 3) kleiner als im Frontalschnitt (Fig. 1) ist.

3. Bandscheibenendoprothese nach Anspruch 1 oder 2, dadurch gekennzeichnet, daß der Krümmungsradius in der einen Hauptrichtung um den Faktor 1,2 bis 2,5 (vorzugsweise 1,5 bis 2) größer ist als in der quer dazu verlaufenden Hauptrichtung.

4. Bandscheibenendoprothese nach einem der Ansprüche 1 bis 3, dadurch gekennzeichnet, daß die Krümmungsbögen der Gelenkfläche (6) sowohl im Medianschnitt als auch im Frontalschnitt im wesentlichen Kreisbögen sind.

5. Bandscheibenendoprothese nach einem der Ansprüche 1 bis 4, dadurch gekennzeichnet, daß der Prothesenkern (3) und die damit über eine Gelenkfläche (6) verbundene(n) Prothesenplatte(n) mit zusammenwirkenden Anschlägen zur Begrenzung der Schwenkbewegung versehen sind.

6. Bandscheibenendoprothese nach Anspruch 5, dadurch gekennzeichnet, daß der Prothesenkern (3) und die Prothesenplatte(n) (1, 2) mit wenigstens einer Nut (12, 14) und einer darin geführten Feder (Rippe 11, 13) versehen sind, die in der Hauptbewegungsrichtung verlaufen und mit Rotationsspiel zueinander ausgerüstet sind.

7. Bandscheibenendoprothese nach Anspruch 5, dadurch gekennzeichnet, daß der Prothesenkern (3) oder die Prothesenplatte(n) (1, 2) einen den Rand (8) des jeweils anderen Teils mit Rotationsspiel umfassenden Kragen (7) aufweist (aufweisen).

8. Bandscheibenendoprothese nach einem der Ansprüche 1 bis 5, dadurch gekennzeichnet, daß die Prothesenplatten (1, 2) mit zusammenwirkenden Anschlägen zur Begrenzung der Schwenkbewegung versehen sind.

## Claims

1. An intervertebral disc endoprosthesis with two prosthesis plates (1,2) which are to be connected with end plates of the associated vertebral bodies, and with a prosthesis core (3) which co-operates with at least one prosthesis plate (1,2) via an articulation surface (6) allowing a rotating movement (10) about the vertical axis (9), characterised in that the articulation surface (6) forms in the central median section and in the central frontal section arcs of curvature of different radius.

2. An intervertebral disc endoprosthesis according to Claim 1, characterised in that the radius of curvature in the median section (Figure 3) is smaller than in the frontal section (Figure 2).

3. An intervertebral disc endoprosthesis according to Claim 1 or 2, characterised in that the radius of curvature in one principal direction is greater by a factor of 1.2 to 2.5 (preferably 1.5 to 2) than in the principal direction extending transversely thereto.

4. An intervertebral disc endoprosthesis according to any one of Claims 1 to 3, characterised in that the arcs of curvature of the articulation surface (6) are substantially circular arcs both in median section and in frontal section.

5. An intervertebral disc endoprosthesis according to any one of Claims 1 to 4, characterised in that the prosthesis core (3) and the prosthesis plate(s) connected therewith via the articulation surface (6) are provided with co-operating stop members to limit the pivotal movement.

6. An intervertebral disc endoprosthesis according to Claim 5, characterised in that the prosthesis core (3) and the prosthesis plate(s) (1,2) are provided with a least one groove (12,14) and one key (rib 11,13) guided therein, which extend in the direction of principal movement and are provided with rotational play relative to one another.

7. An intervertebral disc endoprosthesis according to Claim 5, characterised in that the prosthesis core (3) or the prosthesis plate(s) (1,2) has/have a collar (7) enveloping the rim (8) of the respective other part with rotational play.

8. An intervertebral disc endoprosthesis according to any one of Claims 1 to 5, characterised in that the prosthesis plates (1,2) are provided with co-operating stop members for limiting the pivotal movement.

## Revendications

1. Endoprothèse de disque intervertébral, comprenant deux plaques de prothèse (1,2) qui sont destinées à être fixées aux plaques terminales des corps vertébraux associés et un noyau de prothèse (3) qui coopère avec l'une au moins des plaques de prothèse (1,2) par une surface articulaire (6) permettant un mouvement de rotation (10) autour de l'axe vertical (9), caractérisée en ce que la surface articulaire (6) forme, dans la coupe latérale axiale et dans la coupe frontale axiale, des arcs de courbure de rayons différents.

2. Endoprothèse de disque intervertébral selon la revendication 1, caractérisée en ce que le rayon de courbure dans la coupe latérale (fig. 3) est plus petit que dans la coupe frontale (fig. 1).

3. Endoprothèse de disque intervertébral selon la revendication 1 ou 2, caractérisée en ce que le rayon de courbure dans l'une des directions principales est supérieur d'un facteur de 1,2 à 2,5 (de préférence de 1,5 à 2) au rayon dans la direction principale perpendiculaire à celle-ci.

4. Endoprothèse de disque intervertébral selon l'une quelconque des revendications 1 à 3, caractérisée en ce que les arcs de courbure de la surface articulaire (6) sont essentiellement des arcs de cercle, aussi bien dans la coupe latérale que dans la coupe frontale.

5. Endoprothèse de disque intervertébral selon l'une quelconque des revendications 1 à 4, caractérisée en ce que le noyau de prothèse (3) et la plaque ou les plaques de prothèse reliée(s) à celui-ci par la surface articulaire (6) sont munis de butées qui coopèrent pour limiter le mouvement pivotant.

6. Endoprothèse de disque intervertébral selon la revendication 5, caractérisée en ce que le noyau de prothèse (3) et la ou les plaques de prothèse (1,2) sont munis d'au moins une rainure (12,14) et d'une languette (nervure 11,13) guidée dans celle-ci, qui s'étendent dans la direction de mouvement principale et qui présentent entre elles du jeu à la rotation.

7. Endoprothèse de disque intervertébral selon la revendication 5, caractérisée en ce que le noyau de prothèse (3) ou la ou les plaques de prothèse (1,2) présente(nt) une collerette (7) qui entoure le bord (8) de l'autre élément avec du jeu à la rotation.

8. Endoprothèse de disque intervertébral selon l'une quelconque des revendications 1 à 5, caractérisée en ce que les plaques de prothèse (1,2) sont munies de butées qui coopèrent pour limiter le mouvement pivotant.
